# EUROPEAN PATENT SPECIFICATION

(11) **EP 2 176 235 B1**
(45) Date of publication and mention of the grant of the patent: **18.04.2012**
(21) Application number: 08773249.1
(22) Date of filing: 08.07.2008
(51) Int. Cl.: C07D 235/18

(54) **A METHOD OF MANUFACTURING 4'-[[4-METHYL-6-(1-METHYL-1H-BENZIMIDAZOL-2-YL)-2-PROPYL-1H-BENZIMIDAZOL-1YL]METHYL]BIPHENYL-2-CARBOXYLIC ACID (TELMISARTAN)**
VERFAHREN ZUR HERSTELLUNG VON 4'-[[4-METHYL-6-(1-METHYL-1H-BENZIMIDAZOL-2-YL)-2-PROPYL-1H-BENZIMIDAZOL-1-YL]METHYL]BIPHENYL-2-CARBONSÄURE (TELMISARTAN)
PROCÉDÉ DE FABRICATION D'ACIDE 4'-[[4-MÉTHYL-6-(1-MÉTHYL-1<I>H</I>-BENZIMIDAZOL-2-YL)-2-PROPYL-1<I>H</I>-BENZIMIDAZOL-1-YL]MÉTHYL]BIPHENYL-2-CARBOXYLIQUE (TELMISARTAN)

(30) Priority: 09.07.2007 CZ 20070457
(43) Date of publication of application: 21.04.2010
(73) Proprietor: Zentiva, k.s., Dolni Mecholupy 102 37 Praha 10 (CZ)
(72) Inventor: STACH, Jan, 190 16 Praha 9 (CZ); RADL, Stanislav, 250 84 Kvetnice (CZ); CINIBULK, Josef, 190 00 Praha 9 (CZ); STRELEC, Ivo, 142 00 Praha 4 (CZ); JARRAH, Kamal, 106 00 Praha 10 (CZ)
(74) Representative: Jirotkova, Ivana
(86) International application number: PCT/CZ2008/000080
(87) International publication number: WO 2009/006860

(56) References cited:
- WO-A-2006/044648
- WO-A-2006/044754
- US-B1- 6 358 986

## Description

### Technical Field

The invention deals with an improved method of manufacturing 4'-[[4-methyl-6-(1-methyl-1*H*-benzimidazol-2-yl)-2-propyl-1*H*-benzimidazol-1yl]methyl]biphenyl-2-carboxylic acid (telmisartan) (I)

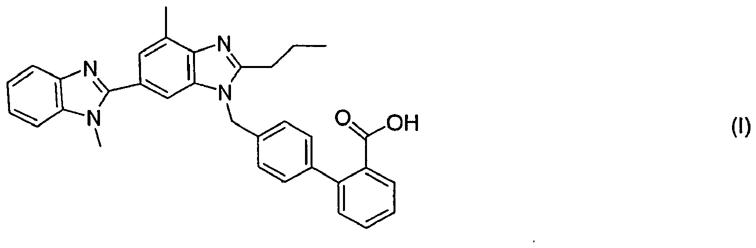

Telmisartan belongs to the group of angiotensin II antagonists, which are being therapeutically used as medicaments for the cardiovascular system, especially to control high blood pressure. A dosage form of telmisartan was introduced in the market in 1998 by Boehringer Ingelheim under the protected name Micardis^{R}. This group contains important drugs like losartan (Cozaar^{R}), irbesartan (Avapro^{R}), or valsartan (Diovan^{R}). However, unlike these substances telmisartan shows better efficiency even in the last hours of the administration interval.

### Background Art

Telmisartan (I) is produced in accordance with the original patent of Boehringer Ingelheim (US 5 591 762) from telmisartan *tert*-butyl ester (II). The hydrolysis is carried out using of trifluoroacetic acid in the toxic solvent *N*,*N*-dimethylformamide.

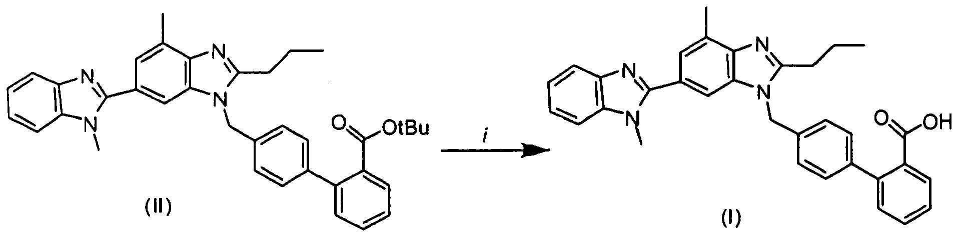

According to another patent applied by the same company (US 2004 236113) the manufacture was problematic and this is why this procedure was replaced with hydrolysis of the corresponding nitrile (III). However, during the hydrolysis, which is carried out with potassium hydroxide in ethylene glycol, a high temperature (160 °C) is used, which causes browning of the product, which must be subsequently purified by means of activated carbon. Also, the energy demands of several-ton production would be considerably high.

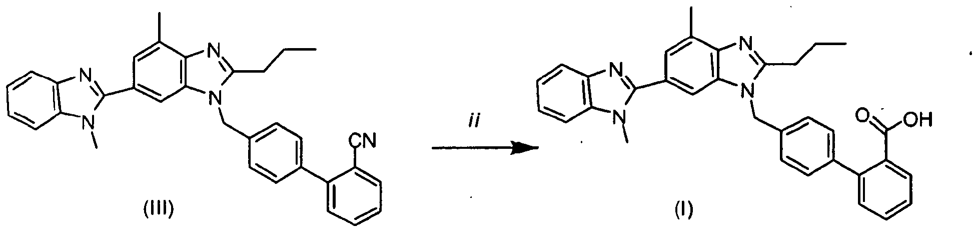

In a newer application of Cipla (WO 2005/108375) the last two synthetic steps (*iii+iv*) are combined and telmisartan is isolated after alkaline hydrolysis by acidifying of the reaction mixture in water or extraction with dichloromethane and precipitation with acetone. Both the ways of isolation are unsuitable for industrial production. In the case of telmisartan of crystalline form A its isolation from water or aqueous solutions of organic solvents is very difficult since a hardly filterable product is formed. Extraction of the product with dichloromethane and precipitation with acetone brings a well-filterable product, but the use of dichloromethane is virtually impossible from the point of view of environment protection.

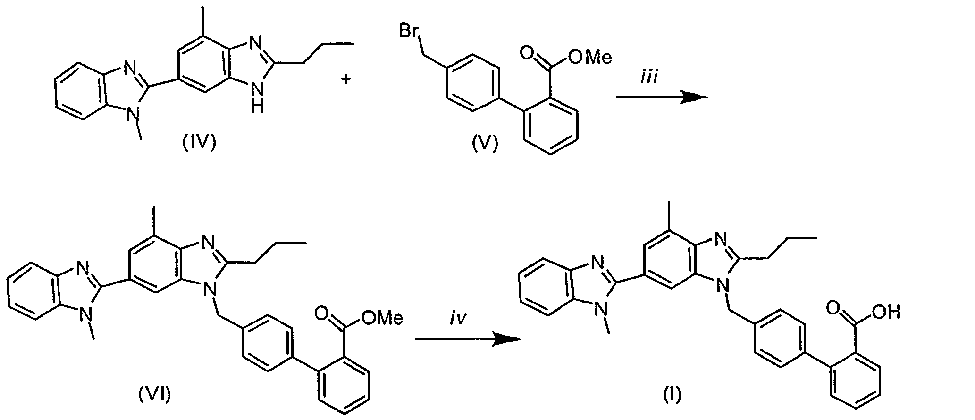

Another method has been described by Dr. Reddy (WO 2006/044754), which starts from telmisartan methylester hydrochloride, which is hydrolyzed to produce the potassium salt of termisartan, which is further acidified in aqueous acetonitrile; after isolation it crystallizes from a dichloromethane/methanol mixture and finally from methanol alone, and wherein a pressure apparatus is used for the dissolution in methanol at a temperature above its boiling point (80 °C). The result of this complex procedure, which manifests the already above mentioned shortcomings, is a low yield of the product.

The method of Teva (WO 2006/044648) is in many aspects similar to the above mentioned procedure of Cipla, wherein the last two steps of the synthesis are also combined. The method comprises phase separations, which lead to low yields (69 % - 80 %) besides increased tediousness.

Matrix starts from telmisartan tert-butyl ester (II), which is first converted to telmisartan dihydrochloride, which in turn, by action of aqueous ammonia in methanol, provides telmisartan with a low total yield of 73%.

### Disclosure of Invention

The object of the invention is an improved method of manufacturing 4'-[[4-methyl-6-(1-methyl-1*H*-benzimidazol-2-yl)-2-propyl-1*H*-benzimidazol-1yl]methyl]biphenyl-2-carboxylic acid (telmisartan) (I). The essence consists in the surprising finding that filterability of the crystalline form A of telmisartan in alcohols depends on the content of water and hence its isolation from an anhydrous solvent is best. Moreover, it has been surprisingly found out that filterability can be improved by the presence of potassium salts of carboxylic acids, which further significantly increases the yield of the process. In addition, the use of carboxylic acids for obtaining telmisartan from its potassium salt provides very good purity of the product.

A detailed description of the invention follows:

Boehringer Ingelheim have described, n US patent 6 410 742, preparation of the new polymorph B, which is said to have better filterability than that of the originally described form A. However, the comparison experiment comprises crystallization from ethanol, wherein telmisartan is converted to an ammonium salt by means of aqueous ammonia and then telmisartan crystallizes by addition of acetic acid. Thus, during crystallization the system contains 2.5% of water, which substantially impairs filterability. In our case it has been surprisingly found out that the content of water in ethanol is of key importance for filterability of the reaction mixture. It has been established that filterability strongly depends on the content of water as well as inorganic salts. An experiment was performed wherein the time of filtration of the product was measured under the same conditions in dependence on the content of water and content of inorganic salts (the end of filtration is measured as disappearance of the solvent phase over aspirated crystals) (Table 1). Crystallization was carried out by addition of acetic acid or formic acid to an ethanolic or methanolic solution of the ammonium or potassium salt of telmisartan in accordance with US patent 6 410 742.

| Weight of telmisartan | Solvent | Water content | Inorganic salt content (salt /telmisartan) | Filtration time (minutes) | Yield |
|---|---|---|---|---|---|
| 10 g | ethanol | 2.5 % (US 6410742) | 23 % (ammonium acetate) | 10 | 90% |
| 10 g | ethanol | 10% | 23 % (ammonium acetate) | 155 | 91% |
| 10g | ethanol | 1% | 23 % (ammonium acetate) | 5 | 89% |
| 10g | ethanol | 0.1% | 23 % (ammonium acetate) | 2 | 87% |
| 10 g | methanol | 2.5 % | 23 % (ammonium acetate) | 11 | 91 % |
| 10 g | methanol | 1 % | 66 % (potassium acetate) | 2 | 95 % |
| 10 g | methanol | 1 % | 90 % (potassium acetate) | 2 | 97 % |
| 10 g | methanol | 0.5 % | 66 % (potassium formate) | 1 | 95 % |

The table shows that in the industrial scale the amount of water and inorganic salts will be the key parameter of the process. The amount of water has a principal impact on filterability of the product and an increased quantity of potassium salts of carboxylic acids reduces solubility of telmisartan and hence has a positive impact on the yield of the process. If the preparation of telmisartan starts from the corresponding methylester, it is also essential to get a product that does not contain inorganic substances. Therefore the inorganic salts used must display high solubility in the alcohols used.

Accordingly, the present invention provides a method of manufacturing the crystalline form A of telmisartan, in which a carboxylic acid of the general formula R¹COOH, wherein R¹ is the hydrogen atom or a C₁-C₄ alkyl, is added to a solution of the potassium salt of telmisartan in an alcohol of the formula R²OH with the water content lower than 2%, wherein R² is ethyl or methyl.

Preferably, the potassium salt of telmisartan is obtained by hydrolysis of a telmisartan alkyl ester of the general formula (VIII) 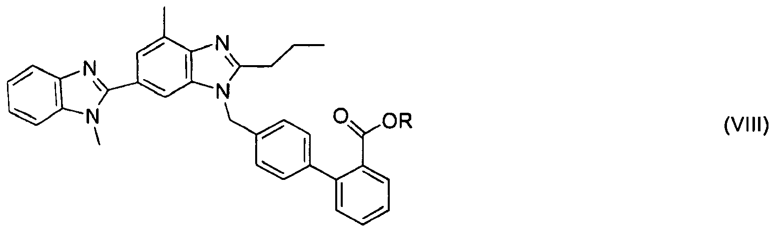
wherein R is methyl or ethyl, with potassium hydroxide in an alcohol of the formula R²OH, wherein R² is ethyl or methyl.

It is convenient if the potassium salt of telmisartan is obtained by neutralization of telmisartan with potassium hydroxide.

Preferably, the content of the obtained potassium salt of the carboxylic acid R¹COOH, expressed as the salt/telmisartan weight ratio, is 20% - 150% during crystallization.

The method according to claim 1, characterized in that acetic acid or formic acid is used as the carboxylic acid.

Preferably, the content of water in the system is lower than 1%.

In one embodiment, the invention provides a method of manufacturing the crystalline form A of telmisartan, characterized in that a telmisartan ester (VIII) is heated up in methanol with the water content lower than 1% by weight together with potassium hydroxide to the boiling temperature for 12 to 48 hours, formic or acetic acid is added to the solution and after cooling the crystalline product of form A is separated.

Preferably, telmisartan of form A is crystallized at a temperature of -10 to +10 °C.

It is convenient if the ratio of telmisartan, potassium hydroxide and formic or acetic acid is selected so as to produce the resulting weight ratio of the potassium salt of the selected organic acid to the resulting telmisartan of 1 : 2 to 6 : 5.

In the course of experiments it has been found that the hydrolysis of telmisartan methylester can be most suitably carried out with potassium hydroxide in anhydrous methanol; after the reaction is complete, the crystalline form A of telmisartan is obtained by addition of acetic or formic acids. Although the product contains a considerable quantity of potassium acetate or formate, it has been found out that the reaction provides the product with a low content of potassium acetate or formate expressed by a low content of sulfate ash. Such mode of carrying out the reaction then complies with the requirements for a synthesis carried out in an industrial scale.

The invention will be elucidated in a more detailed way in the following examples. These examples, which illustrate the improvement of the procedure according to the invention, are of an illustrative character only and do not limit the scope of the invention in any aspect.

### Examples

### Example 1

### 4'-[[4-methyl-6-(1-methyl-1H-benzimidazol-2-yl)-2-propyl-1H-benzimidazol-1 yl]methyl]biphenyl-2-carboxylic acid (telmisartan)

Telmisartan methylester (VI) (40 g) was refluxed in methanol (440 ml) with potassium hydroxide (14.9 g) for 24 hours. To the boiling solution, methanol (240 ml) and then acetic acid (45.5 g) were added. While boiling, the mixture was stirred for another 1 hour, after cooling to 4°C the product was aspirated within 1 hour and washed with methanol (2 x 80 ml). After drying at the laboratory temperature (24 h) 35.18 g (90 %) of the product were obtained.

### Analytic assessment:

### HPLC purity: 99.90 %,

| | |
|---|---|
| Content of residual solvents: | methanol (below the detection limit) acetic acid (360 ppm) |

Titration content: 100.9 %
Sulfate ash content: 0.04 %
DSC: form A

### Example 2

### 4'-[[4-Methyl-6-(1-methyl-1H-benzimidazol-2-yl)-2-propyl-1H-benzimidazol-1yl]methyl]biphenyl-2-carboxylic acid (telmisartan)

Telmisartan methylester (VI) (20 g) was refluxed in methanol (300 ml) with potassium hydroxide (7 g) for 24 h. After addition of formic acid (17 g) and after cooling to 4 °C the product was aspirated within 1 hour and washed with methanol (2 x 80 ml). After drying at the laboratory temperature (24 h) 18.7 g (96 %) of the product were obtained.

### Example 3

### 4'-[[4-methyl-6-(1-methyl-1H-benzimidazol-2-yl)-2-propyl-1H-benzimidazol-1yl]methyl]biphenyl-2-carboxylic acid (telmisartan)

Telmisartan methylester (VI) (20 kg) was refluxed in methanol (400 1) with potassium hydroxide (7 kg) for 24 h. After addition of acetic acid (20 kg) and cooling to 4 °C the product was aspirated within 1 hour and washed with methanol (2 x 80 1). After drying at the laboratory temperature (24 h) 18.5 kg (95 %) of the product were obtained.

### Example 4

### 4'-[[4-methyl-6-(1-methyl-1H-benzimidazol-2-yl)-2-propyl-1H-benzimidazol-1 yl]methyl]biphenyl-2-carboxylic acid (telmisartan)

Telmisartan methylester (40 g) was refluxed in methanol (240 ml) with potassium hydroxide (14.9 g) for 24 h. To the boiling solution methanol (240 ml) and then acetic acid (45.5 g) were added. After cooling to 4 °C the product was aspirated within 1 hour and washed with methanol (2 x 80 ml). After drying at the laboratory temperature (24 h) 36 g (92%) of the product were obtained.

## Claims

1. A method of manufacturing the crystalline form A of telmisartan (I) 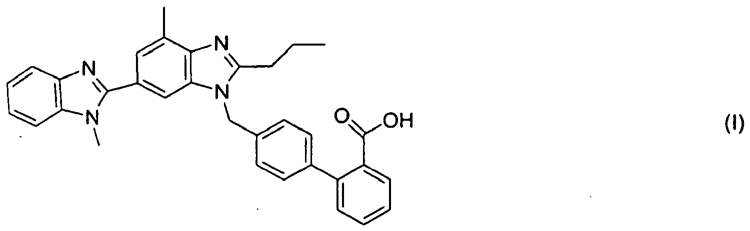 **characterized in that** a carboxylic acid of the general formula R¹COOH, wherein R¹ is the hydrogen atom or a C₁-C₄ alkyl, is added to a solution of the potassium salt of telmisartan (VII) 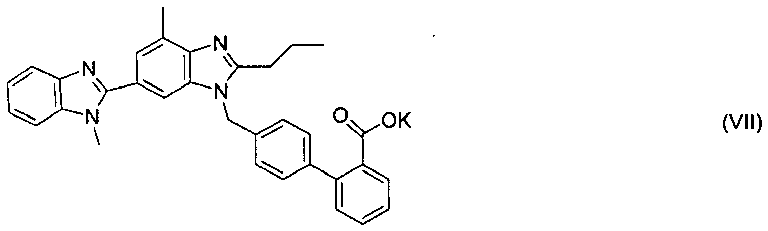 in an alcohol of the formula R²OH with the water content lower than 2%, wherein R² is ethyl or methyl.

2. The method according to claim 1, **characterized in that** the potassium salt of telmisartan is obtained by hydrolysis of a telmisartan alkyl ester of the general formula (VIII), 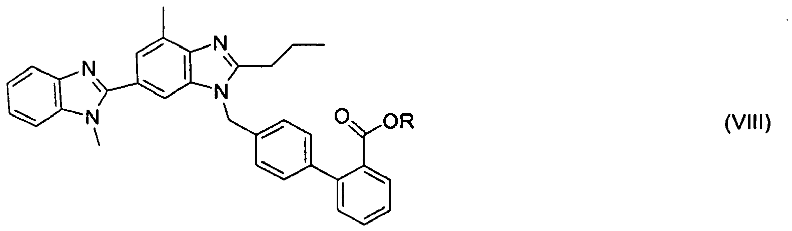 wherein R is methyl or ethyl, with potassium hydroxide in an alcohol of the formula R²OH, wherein R² is ethyl or methyl.

3. The method according to claim 1, **characterized in that** the potassium salt of telmisartan is obtained by neutralization of telmisartan of formula (I) with potassium hydroxide.

4. The method according to claim 1, **characterized in that** the content of the obtained potassium salt of the carboxylic acid R¹COOH, expressed as the salt/telmisartan weight ratio, is 20% - 150% during crystallization.

5. The method according to claim 1, **characterized in that** acetic acid or formic acid is used as the carboxylic acid.

6. The method according to claim 1, **characterized in that** the content of water in the system is lower than 1%.

7. A method of manufacturing the crystalline form A of telmisartan (I), **characterized in that** a telmisartan ester (VIII) is heated up in methanol with the water content lower than 1% by weight together with potassium hydroxide to the boiling temperature for 12 to 48 hours, formic or acetic acid is added to the solution and after cooling the crystalline product of form A is separated.

8. The method according to claim 7, **characterized in that** telmisartan of form A is crystallized at a temperature of -10 to + 10°C.

9. The method according to claim 7, **characterized in that** the ratio of telmisartan, potassium hydroxide and formic or acetic acid is selected so as to produce the resulting weight ratio of the potassium salt of the selected organic acid to the resulting telmisartan of 1 : 2 to 6 : 5.

## Patentansprüche

1. Verfahren zur Herstellung der kristallinen Form A von Telmisartan (I) 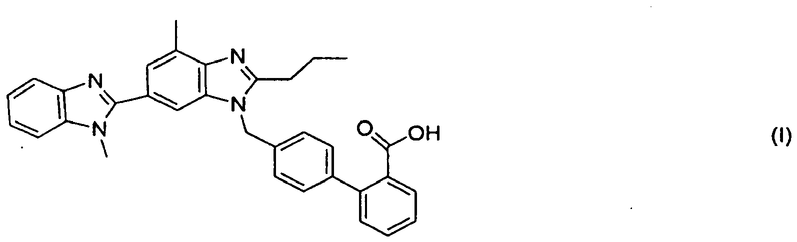 **dadurch gekennzeichnet, dass** eine Carbonsäure der allgemeinen Formel R¹COOH, in welcher R¹ das Wasserstoffatom oder ein C₁-C₄-Alkyl ist, zu einer Lösung des Kaliumsalzes von Telmisartan (VII) 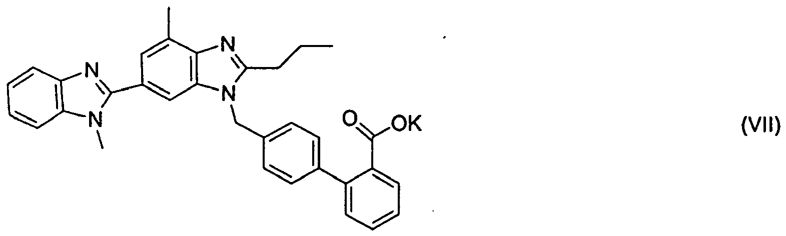 in einem Alkohol der Formel R²OH mit einem Wassergehalt von weniger als 2 % hinzugegeben wird, wobei R² Ethyl oder Methyl ist.

2. Verfahren nach Anspruch 1, **dadurch gekennzeichnet, dass** das Kaliumsalz von Telmisartan durch Hydrolyse eines Telmisartanalkylesters der allgemeinen Formel (VIII) 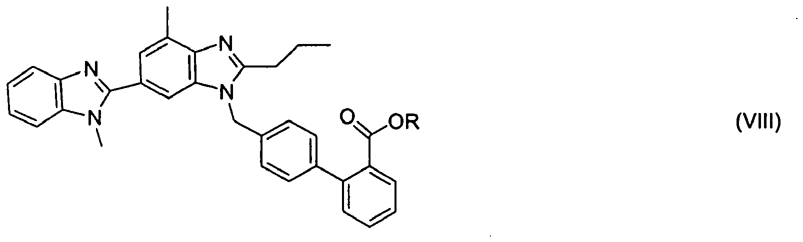 in welcher R Methyl oder Ethyl ist, mit Kaliumhydroxid in einem Alkohol der Formel R²OH, in welcher R² Ethyl oder Methyl ist, erhalten wird.

3. Verfahren nach Anspruch 1, **dadurch gekennzeichnet, dass** das Kaliumsalz von Telmisartan durch Neutralisieren von Telmisartan der Formel (I) mit Kaliumhydroxid erhalten wird.

4. Verfahren nach Anspruch 1, **dadurch gekennzeichnet, dass** der Gehalt an dem erhaltenen Kaliumsalz der Carbonsäure R¹COOH, ausgedrückt als Salz/Telmisartan-Gewichtsverhältnis, während der Kristallisation 20 % - 150 % beträgt.

5. Verfahren nach Anspruch 1, **dadurch gekennzeichnet, dass** als Carbonsäure Essigsäure oder Ameisensäure verwendet wird.

6. Verfahren nach Anspruch 1, **dadurch gekennzeichnet, dass** der Wassergehalt in dem System weniger als 1 % beträgt.

7. Verfahren zur Herstellung der kristallinen Form A von Telmisartan (I), **dadurch gekennzeichnet, dass** ein Telmisartanester (VIII) in Methanol mit einem Wassergehalt von weniger als 1 Gew.-% zusammen mit Kaliumhydroxid für einen Zeitraum von 12 bis 48 Stunden auf Siedetemperatur erhitzt, die Lösung mit Ameisensäure oder Essigsäure versetzt wird und nach Abkühlen das kristalline Produkt der Form A abgetrennt wird.

8. Verfahren nach Anspruch 7, **dadurch gekennzeichnet, dass** Telmisartan der Form A bei einer Temperatur von -10 bis +10 °C kristallisiert wird.

9. Verfahren nach Anspruch 7, **dadurch gekennzeichnet dass** das Verhältnis von Telmisartan, Kaliumhydroxid und Ameisen- oder Essigsäure so gewählt ist, dass sich ein Gewichtsverhältnis des Kaliumsalzes der gewählten organischen Säure zu dem erhaltenen Telmisartan von 1 : 2 bis 6 : 5 ergibt.

## Revendications

1. Un procédé de fabrication de la forme cristalline A du telmisartan (I) 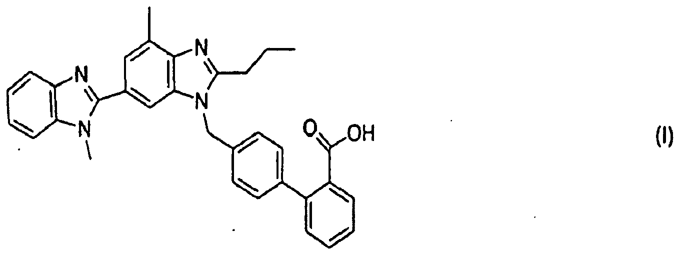 **caractérisé en ce qu'**un acide carboxylique de formule générale R¹COOH, dans laquelle R¹ est un atome d'hydrogène ou un radical (C₁-C₄)-alkyl, est ajouté à une solution du sel de potassium de telmisartan (VII) 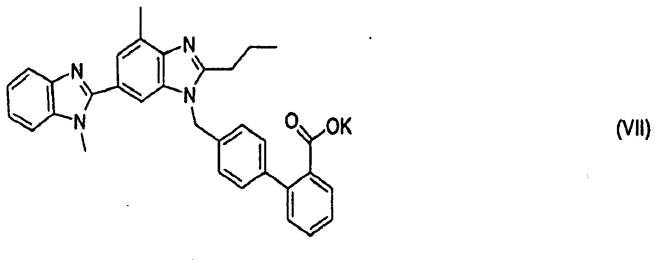 dans un alcool de formule R²OH présentant une teneur en eau inférieure à 2%, R² étant un radical éthyle ou méthyle.

2. Le procédé selon la revendication 1, **caractérisé en ce que** le sel de potassium de telmisartan est obtenu par hydrolyse d'un ester alkylique de telmisartan présentant la formule générale (VIII), 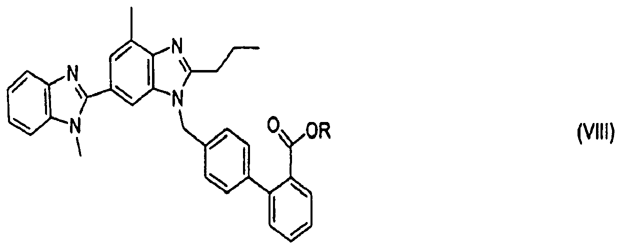 dans laquelle R est un radical méthyle ou éthyle, avec l'hydroxyde de potassium, dans un alcool de formule R²OH, où R² est un radical éthyle ou méthyle.

3. Le procédé selon la revendication 1, **caractérisé en ce que** le sel de potassium de telmisartan est obtenu par neutralisation de telmisartan de formule (I) avec un hydroxyde de potassium.

4. Le procédé selon la revendication 1, **caractérisé en ce que** la proportion de sel de potassium de l'acide carboxylique R¹COOH obtenue, durant la cristallisation, exprimée sous forme du rapport pondéral sel/telmisartan, est de 20% - 150%.

5. Le procédé selon la revendication 1, **caractérisé en ce qu'**est employé en tant qu'acide carboxylique l'acide acétique ou l'acide formique.

6. Le procédé selon la revendication 1, **caractérisé en ce que** la teneur en eau du système est inférieure à 1%.

7. Un procédé de fabrication de la forme cristalline A du telmisartan (I), **caractérisé en ce qu'**un ester de telmisartan (VIII) est réchauffé dans du méthanol dont la teneur en eau est inférieure à 1 % en poids, en présence d'un hydroxyde de potassium, à la température d'ébullition durant 12 à 48 heures, **en ce qu'**est ajouté à la solution de l'acide formique ou de l'acide acétique et **en ce que**, après refroidissement, est séparé le produit cristallin de forme A.

8. Le procédé selon la revendication 7, **caractérisé en ce que** le telmisartan de forme A est cristallisé à une température comprise entre -10°C et +10°C.

9. Le procédé selon la revendication 7, **caractérisé en ce que** le rapport telmisartan/hydroxyde de potassium/acide formique ou acétique est choisi de façon à ce qu'il produise le rapport pondéral sel de potassium de l'acide organique choisi/telmisartan résultant de 1/2 à 6/5.
